# EUROPEAN PATENT APPLICATION

(11) **EP 4 067 375 A1**
(43) Date of publication of application: **05.10.2022**
(21) Application number: 21165998.2
(22) Date of filing: 30.03.2021
(51) Int. Cl.: C07K 14/54, A61K 38/00

(54) **FIBRIL PEPTIDES**

(71) Applicant: Universität Ulm, 89081 Ulm (DE)
(72) Inventor: Jan, Münch, 89233 Neu-Ulm (DE); Schütz, Desiree, 89198 Westerstetten (DE); Rauch, Lena, 88410 Bad Wurzach (DE)
(74) Representative: Altmann Stößel Dick Patentanwälte PartG mbB

(57) **Abstract**

The present invention relates to a fibril peptide consisting of a sequence of from 8 to 20 amino acids and wherein said fibril peptide comprises the amino acid sequence RTIFIISM or a sequence at least 75% identical thereto. The present invention further relates to a method of delivering a cargo comprised in a particle into a host cell, comprising a) contacting said host cell with said particle and with a fibril peptide according to any one of claims 1 to 8; and, thereby b) delivering said cargo into said host cell; and to uses, kits, and devices related thereto.

## Description

The present invention relates to a fibril peptide consisting of a sequence of from 8 to 20 amino acids and wherein said fibril peptide comprises the amino acid sequence RTIFIISM or a sequence at least 75% identical thereto. The present invention further relates to a method of delivering a cargo comprised in a particle into a host cell, comprising a) contacting said host cell with said particle and with a fibril peptide according to any one of claims 1 to 8; and, thereby b) delivering said cargo into said host cell; and to uses, kits, and devices related thereto.

One of the basic requirements of gene technology is the ability to introduce nucleic acids into target cells at high efficiency. For this purpose, a number of technologies have been developed, e.g. introduction of naked DNA or vector systems such as viruses, of which retroviruses are widely used for their broad host cell spectrum and their stable integration into the target cell genome . Nonetheless, further improvement of virus or viral vector infection frequently is desirable. For this purpose, e.g. cationic polymers such as DEAE-dextran, recombinant proteins such as RetroNectin^{®}, and peptide-based enhancer have been used.

Peptide transduction enhancers have the advantage that they are biodegradable, and their small size makes an easy characterization and cheap production possible. Protamine sulfate is a mixture of highly cationic peptides derived from the sperm of different fish species, which enhance retroviral infection in a similar way such as cationic polymers. Protamine sulfate is due to its lower cytotoxicity an effective alternative to polybrene (Cornetta et al., *J*. *Virol. Methods* 1989, 23 (2), 187). Not only the peptide as monomer lead to amplification of transduction, but also nanofibril-forming peptides such as Vectofusin-1, semen derived enhancer of viral infection (SEVI) and enhancing factor C (EF-C).

Vectofusin-1 forms pH-dependent α-helical nanofibrils. It assembles spontaneously in culture medium to supramolecular structures, which leads to the co-localization of viral particles. This results in sedimentation of the virus particles along the cellular surface of the target cell (Majdoul et al., J. Biol. Chem. 2016, 291 (5), 2161). Other peptides such as SEVI and EF-C form β-sheet nanofibrils. SEVI is the name of fibrils that arise from the prostatic acid phosphatase (PAP), which encompasses the amino acid residues 248-286. SEVI fibrils have a diameter of 5.3±0.1 µm and a length of 5-10 µm (Munch et al., Cell 2007, 131 (6), 1059). These fibrils capture human immunodeficiency virus (HIV) virions and promote the attachment to target cells, which enhance viral infection. At physiological concentrations SEVI fibrils amplify HIV infection of different cells e.g. T cells and macrophages. But they also increase gene transfer by γ-RV and LV into hematopoietic and non-hematopoietic cells. Also for VSV-G pseudotyped viral vectors this beneficial effect can be seen (Wurm et al., J. Gene Med. 2010, 12 (2), 137). However, the limiting factor is the expensive production of the relatively large 39-amino acid residue peptide and the fibril formation takes at least 2 h. Additionally extended storage of SEVI can lead to the formation of larger aggregates with lower enhancing activity. EF-C (Protransduzin^{®}-A, EP 2 452 947 A1) and Protransduzin^{®}-B (US 2016/0185820) are amphiphilic 12-amino acid peptides derived from HIV-1 glycoprotein gp120. EF-C forms β-amyloid nanofibrils which have a diameter of 3.4 ± 1.1 nm, a length in the range of 100-400 nm and a twist with a half-pitch of 26 ± 2 nm. A model of EF-C nanofibrils demonstrates that at physiological pH the lysine side chains form a hydrophilic surface with a high density of positive charges (Yolamanova et al, Nat. Nanotechnol. 2013, 8 (2), 130).

There is, nonetheless, a continued need for improved methods to introduce polynucleotides into host cells.

The above problem is addressed by the compounds, methods, uses, kits, and devices with features of the independent claims. Advantageous embodiments which might be realized in an isolated fashion or in any arbitrary combinations are listed in the dependent claims.

In accordance, the present invention relates to a fibril peptide consisting of a sequence of from 8 to 20 amino acids and wherein said fibril peptide comprises the amino acid sequence RTIFIISM or a sequence at least 75% identical thereto.

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way.

Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one".

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise noted, relates to IUPAC standard ambient temperature and pressure (SATP) conditions, i.e. preferably, a temperature of 25°C and an absolute pressure of 100 kPa; also preferably, standard conditions include a pH of 7. Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1% by weight, most preferably less than 0.1% by weight of non-specified component(s).

The degree of identity (e.g. expressed as "%identity") between two biological sequences, preferably DNA, RNA, or amino acid sequences, can be determined by algorithms well known in the art. Preferably, the degree of identity is determined by comparing two optimally aligned sequences over a comparison window, where the fragment of sequence in the comparison window may comprise additions or deletions (e.g., gaps or overhangs) as compared to the sequence it is compared to for optimal alignment. The percentage is calculated by determining, preferably over the whole length of the polynucleotide or polypeptide, the number of positions at which the identical residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. In the context of biological sequences referred to herein, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis.

The term "fragment" of a biological macromolecule, preferably of a polynucleotide or polypeptide, is used herein in a wide sense relating to any sub-part, preferably subdomain, of the respective biological macromolecule comprising the indicated sequence, structure and/or function. Thus, the term includes sub-parts generated by actual fragmentation of a biological macromolecule, but also sub-parts derived from the respective biological macromolecule in an abstract manner, e.g. in silico. Thus, as used herein, an Fc or Fab fragment, but also e.g. a single-chain antibody, a bispecific antibody, and a nanobody may be referred to as fragments of an immunoglobulin.

Unless specifically indicated otherwise herein, the compounds specified, in particular the (poly)peptides and polynucleotides, may be comprised in larger structures, e.g. may be covalently or non-covalently linked to each other, to carrier molecules, retardants, and other excipients. In particular, (poly)peptides as specified may be comprised in fusion polypeptides comprising further peptides, which may serve e.g. as a tag for purification and/or detection, as a linker, or to extend the *in vivo* half-life of a compound. The term "detectable tag" refers to a stretch of amino acids which are added to or introduced into the fusion polypeptide; preferably, the tag is added C- or N- terminally to the fusion polypeptide of the present invention. Said stretch of amino acids preferably allows for detection of the fusion polypeptide by an antibody which specifically recognizes the tag; or it preferably allows for forming a functional conformation, such as a chelator; or it preferably allows for visualization, e.g. in the case of fluorescent tags. Preferred detectable tags are the Myc-tag, FLAG-tag, 6-His-tag, HA-tag, GST-tag or a fluorescent protein tag, e.g. a GFP-tag. These tags are all well known in the art. Other further peptides preferably comprised in a fusion polypeptide comprise further amino acids or other modifications which may serve as mediators of secretion, as mediators of blood-brain-barrier passage, as cell-penetrating peptides, and/or as immune stimulants. Further polypeptides or peptides to which the polypeptides may be fused are signal and/or transport sequences, or linker sequences. Preferably, however, the (poly)peptides, in particular the fibril peptides, consist of the amino acids as indicated.

The term "polypeptide", as used herein, refers to a molecule consisting of several, typically at least 20 amino acids that are covalently linked to each other by peptide bonds. Molecules consisting of less than 20 amino acids covalently linked by peptide bonds are usually considered to be "peptides".

The term "fibril peptide", as used herein, relates to a peptide comprising, preferably consisting of, the amino acid sequence RTIFIISM (SEQ ID NO:1) or a sequence at least 75% identical thereto and having the property of forming fibrils in an aqueous solution. Preferably, the fibril peptide comprises, preferably consists of, the amino acid sequence RTIFIISM (SEQ ID NO:1) or a sequence at least 85% identical thereto. Preferably, the fibril peptide has a length of from 8 to 20 amino acids, more preferably of from 8 to 16 amino acids, still more preferably of from 8 to 14 amino acids, even more preferably of from 8 to 12 amino acids, most preferably of from 8 to 10 amino acids. Preferably, the fibril peptide is a cationic peptide, i.e. has a net positive charge, under standard conditions, preferably at a pH of 7. Preferably, the fibril peptide comprises, preferably consists of, the amino acid sequence X¹TIFIISM (SEQ ID NO:2), RX²IFIISM (SEQ ID NO:3), RTX²FIISM (SEQ ID NO:4), RTIX²IISM (SEQ ID NO:5), RTIFX²ISM (SEQ ID NO:6), RTIFIX²SM SEQ ID NO:7), RTIFIIX²M (SEQ ID NO:8), or RTIFIISX² (SEQ ID NO:9), wherein X¹ is a positively charged amino acid, preferably R, K or H, and wherein X² is any amino acid. More preferably, the fibril peptide comprises, preferably consists of, the amino acid sequence X¹TIFIISM (SEQ ID NO:2), RX²IFIISM (SEQ ID NO:3), RTX²FIISM (SEQ ID NO:4), RTIX²IISM (SEQ ID NO:5), RTIFX²ISM (SEQ ID NO:6), RTIFIX²SM SEQ ID NO:7), RTIFIIX²M (SEQ ID NO:8), or RTIFIISX² (SEQ ID NO:9), wherein X¹ is R or K and wherein X is selected from A, V, L, I, M, F, T, W, G, S, T, N, Q, R, H, K, and derivatives thereof. Still more preferably, the fibril peptide comprises, preferably consists of, the amino acid sequence X¹TIFIISM (SEQ ID NO:2), RX²IFIISM (SEQ ID NO:3), RTX²FIISM (SEQ ID NO:4), RTIX²IISM (SEQ ID NO:5), RTIFX²ISM (SEQ ID NO:6), RTIFIX²SM SEQ ID NO:7), RTIFIIX²M (SEQ ID NO:8), or RTIFIISX² (SEQ ID NO:9), wherein X¹ is R or K and wherein X² is selected from the list consisting of A, V, L, I, M, F, T, W, G, S, T, N, Q, R, H, K and non-negatively charged derivatives thereof; preferably wherein X¹ is R or K and wherein X² is selected from the list consisting of A, V, L, I, M, F, T, and non-negatively charged derivatives thereof. Even more preferably, the fibril peptide comprises, preferably consists of, the amino acid sequence X¹TIFIISM (SEQ ID NO:2), RX²IFIISM (SEQ ID NO:3), RTX²FIISM (SEQ ID NO:4), RTIX²IISM (SEQ ID NO:5), RTIFX²ISM (SEQ ID NO:6), RTIFIX²SM SEQ ID NO:7), RTIFIIX²M (SEQ ID NO:8), or RTIFIISX² (SEQ ID NO:9), with X¹ and X² as specified herein above. Still more preferably, the fibril peptide consists of the amino acid sequence RTIFIISMX³X⁴ (SEQ ID NO:10), RAIFIISMX³X⁴ (SEQ ID NO:11), RTAFIISMX³X⁴ (SEQ ID NO: 12), RTIAIISMX³X⁴(SEQ ID NO: 13), RTIFAISMX³X⁴ (SEQ ID NO:14), RTIFIASMX³X⁴ (SEQ ID NO:15), RTIFIIAMX³X⁴ (SEQ ID NO:16), RTIFIISAX³X⁴ (SEQ ID NO: 17), wherein X³ and X⁴ are independently selected from any amino acid or wherein X³ and/or X⁴ are absent; preferably wherein X³ is selected from the list consisting of Y, A, V, L, I, M, F, T, W, G, S, T, N, Q, R, H, K, and derivatives thereof, preferably non-negatively charged derivatives thereof, more preferably wherein X³ is Y; and/or wherein X⁴ is selected from the list consisting of K, R, H, A, V, L, I, M, F, T, W, G, S, T, N, Q, and derivatives thereof, preferably non-negatively charged derivatives thereof, more preferably, wherein X⁴ is K. Preferably, the fibril peptide consists of the amino acid sequence RTIFIISM (SEQ ID NO:1), RAIFIISM (SEQ ID NO:18), RTAFIISM (SEQ ID NO:19), RTIAIISM (SEQ ID NO:20), RTIFAISM (SEQ ID NO:21), RTIFIASM (SEQ ID NO:22), RTIFIIAM (SEQ ID NO:23), RTIFIISA (SEQ ID NO:24), RTIFIISMY (SEQ ID NO:25), RAIFIISMY (SEQ ID NO:26), RTAFIISMY (SEQ ID NO:27), RTIAIISMY (SEQ ID NO:28), RTIFAISMY (SEQ ID NO:29), RTIFIASMY (SEQ ID NO:30), RTIFIIAMY (SEQ ID NO:31), RTIFIISAY (SEQ ID NO:32), RTIFIISMA (SEQ ID NO:33), RTIFIISMYK (SEQ ID NO:34), RAIFIISMYK (SEQ ID NO:35), RTAFIISMYK (SEQ ID NO:36), RTIAIISMYK (SEQ ID NO:37), RTIFAISMYK (SEQ ID NO:38), RTIFIASMYK (SEQ ID NO:39), RTIFIIAMYK (SEQ ID NO:40), RTIFIISAYK (SEQ ID NO:41), RTIFIISMAK (SEQ ID NO:42), or RTIFIISMYA (SEQ ID NO:43), more preferably of the amino acid sequence RTIFIISM (SEQ ID NO:1), RAIFIISM (SEQ ID NO:18), RTAFIISM (SEQ ID NO:19), RTIAIISM (SEQ ID NO:20), RTIFAISM (SEQ ID NO:21), RTIFIASM (SEQ ID NO:22), RTIFIIAM (SEQ ID NO:23), RTIFIISA (SEQ ID NO:24), or RTIFIISMYK (SEQ ID NO:34), preferably RTIFIISM (SEQ ID NO:1) or RTIFIISMYK (SEQ ID NO:34).

The term "derivative" of an amino acid is used herein in a broad sense and includes any and all, naturally occurring or non-naturally occurring, organic acids comprising an amino group and which can be covalently bonded to a peptide via at least one peptide bond. Preferably, the amino acid derivative is an alpha-amino acid, more preferably an L-amino acid, still more preferably is a proteinogenic amino acid. More preferably, the amino acid derivative of an amino acid is a straight-chain amino acid, preferably homoalanine, norvaline, or norleucine; a D-amino acid, preferably D-alanine, D-leucine, or D-isoleucine; a non-alpha amino acid, preferably beta-alanine or gamma-amino-butyric acid; a derivative comprising an exchange of an atom by an atom from the same chemical group, preferably selenomethionine; and/or a naturally occurring derivative of an amino acid, preferably hypusine.

The fibril peptide has the activity of forming fibrils in an aqueous solution, the term "fibril" relating to any threadlike structure or filament comprising, preferably consisting of, a multitude of fibril peptides; preferably, the fibrils are nanofibrils, i.e. have a diameter of from 2 to 20 nm, and/or a length of from 100 nm to 10 µm, preferably of from 100 nm to 5 µm. Preferably, aggregation of fibril peptides occurs spontaneously in aqueous solution, wherein an aqueous solution is a solution comprising at most 25% (v/v), preferably at most 15% (v/v), more preferably at most 10% (v/v), still more preferably at most 5% (v/v), most preferably at most 2% (v/v), organic solvents. Methods for determining the activity of forming fibrils are known to the skilled person and are shown herein in the Examples. Preferably, the activity of forming fibrils is determined by a Thioflavin T binding assay as specified herein in the Examples. The activity of forming fibrils may, however, also be determined by Transmission electron microscopy (TEM), Congo Red staining, measurement of conversion rate, and/or Fourier transform infrared (FTIR) spectroscopy; the aforesaid methods are known in the art.

Preferably, the fibril peptide further has the biological activity of enhancing virus infection of a host cell. Assays for establishing said biological activity are known to the skilled person and are shown herein in the Examples. Preferably, said biological activity is determined by determining the infection efficiency of HIV in TZM-bl cells compared to a control; wherein preferably, the viral tat protein transactivates a β-galactosidase reporter gene under the control of the HIV-1-LTR promoter in TZM-bl cells. Preferably, enhancing virus infection is causing an increase of infection rate compared to a control by a factor of at least 2, more preferably by a factor of at least 5, even more preferably by a factor of at least 10. Preferably, the fibril peptide is used at a concentration of from 0.01 µg/ml to 100 µg/ml, more preferably of from 0.05 µg/ml to 75 µg/ml, even more preferably of from 0.1 µg/ml to 50 µg/ml, still more preferably of from 0.2 µg/ml to 30 µg/ml, even more preferably of from 0.5 µg/ml to 25 µg/ml, most preferably of from 1 µg/ml to 20 µg/ml, for enhancing virus infection.

Preferably, the fibril peptide has low immunogenicity. The term "immunogenicity" is understood by the skilled person. Preferably, the term relates to an induction of a response of the adaptive immune system of a subject, more preferably to a detectable response, most preferably a response causing at least one systemic symptom of an immune reaction in a subject. In accordance, the term "low immunogenicity", as used herein, relates to immune response not causing a response causing at least one systemic symptom of an immune reaction in a subject, more preferably relates to a non-detectable immune response. Thus, preferably, after intramuscular administration of 0.1 µg of fibril peptide/kg body weight to a subject, after four weeks, no peptide-specific antibodies and/or no peptide specific T cells are detectable in case of a peptide having low immunogenicity.

The term "subject", as used herein, relates to an animal or plant, preferably a vertebrate, more preferably a mammal, in particular to livestock like cattle, horse, pig, sheep, and goat, to a companion animal like a dog or cat, or to a laboratory animal like a rat, a mouse, or a guinea pig. Most preferably, the subject is a human.

The fibril peptide may be produced by any method deemed appropriate by the skilled person. Preferably, the fibril peptide is produced by overexpression is a suitable cell, preferably a bacterial or yeast cell, more preferably a bacterial cell, most preferably an E. coli cell. Also preferably, the fibril peptide is produced in a mammalian cell, or an insect cell, preferably in a mammalian cell, more preferably in CHO cells. More preferably, the fibril peptide is produced by chemical synthesis according to methods known in the art. Preferably, the fibril peptide is purified after synthesis, e.g. by chromatography, preferably reverse-phase chromatography, desalting, and/or other methods customary in the art for peptide purification.

Preferably, the fibril peptide is comprised in a pharmaceutical composition. The terms "medicament" and "pharmaceutical composition" are, in principle, known to the skilled person. As referred to herein, the terms relate to any composition containing a fibril peptide and a particle comprising a cargo or salt or prodrug thereof as pharmaceutically active compound and, optionally, one or more other components such as one or more pharmaceutically acceptable excipient(s), including e.g. pharmaceutically acceptable carrier(s). The term "pharmaceutically acceptable excipient" preferably includes any and all material suitable for preparing a pharmaceutical composition to be administered to a living being without being deleterious thereto. Thus, the term includes any solvents, diluents, or other vehicles, dispersion or suspension aids, surface active agents, isotonic agents, thickening or emulsifying agents, preservatives, solid binders, lubricants and the like, as suited to the particular dosage form desired. Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions. The pharmaceutically active compound preferably is present in the medicament in liquid or dry, e.g. lyophilized, form. The pharmaceutically active compound is the active ingredient or drug of the medicament, and is preferably administered in a conventional dosage form prepared by combining the drug with standard pharmaceutical excipients according to conventional procedures. These procedures may involve mixing, granulating, compression, and/or dissolving the ingredients as appropriate to the desired preparation. It will be appreciated that the form and character of the pharmaceutical acceptable carrier or diluent is dictated by the amount of active ingredient with which it is to be combined, the route of administration, and other well-known variables. The excipient(s) must be acceptable in the sense of being compatible with the other ingredients of the formulation and being not deleterious to the recipient thereof. In addition, the pharmaceutical composition or formulation may also include other carriers, adjuvants, preservatives, stabilizers, surfactants, buffers, and/or salts for regulating osmotic pressure, emulsifiers, sweeteners, colorants, flavourings and the like. Preferably, the amount of the compound in the pharmaceutical composition is the amount usually employed for other indications of the specific compound. The amount can vary over a wide range depending on known factors, for example, as specified herein below. However, a person skilled in the art can determine the optimum amount in easy and routine manner, in particular based on the existing dosage recommendations for the compound.

The route of administration of the pharmaceutical composition of the present invention depends on the specific compound used or contained in the pharmaceutical composition and is in accordance with known methods for administration, which can be, for example inhalation, injection or infusion by intravenous, parenterally, topically, rectally, nasally, buccally, vaginally or via an implanted reservoir, or by sustained release systems. The term parenteral as used herein includes intravenous, intraperitoneal, intracerebral, intrathecal, intracranial, intramuscular, intraarticular, intrasynovial, intrasternal, intraocular, intraarterial, subcutaneous, and intracutaneous or intralesional injection or infusion techniques. Preferably, the compound or the pharmaceutical composition is administered such that a concentration sufficient to achieve the effect of treating and/or preventing disease or disorder is achieved, i.e. such that an effective concentration is achieved. Moreover, the composition may be formulated into inhalable or injectable dosage forms such as solutions, suspensions, and emulsions by further adding diluents, dispersants, and surfactants. Further, the composition may be suitably formulated using appropriate methods known in the art or by the method disclosed in Remington's Pharmaceutical Science (recent edition), Mack Publishing Company, Easton Pa.

The pharmaceutical composition is, preferably, administered in conventional dosage forms prepared by combining the active compounds with standard pharmaceutically acceptable excipients and/or carriers as specified herein above and according to conventional procedures. A therapeutically effective dose refers to an amount of the active compound to be used in a pharmaceutical composition which provides the effect referred to herein. Therapeutic efficacy and toxicity of compounds can be determined by standard pharmaceutical procedures in cell cultures or experimental animals, e.g., ED50 (the dose therapeutically effective in 50% of the population) and LD50 (the dose lethal to 50% of the population), or IC50 (the dose causing 50% inhibition). The dose ratio between therapeutic and toxic effects is the therapeutic index, and it can be expressed as the ratio, LD50/ED50. The dosage regimen will be determined by the attending physician, preferably based on clinical factors; preferably in accordance with any one of the above described methods. As is well known in the medical arts, dosages for any one patient depend upon many factors, which may include the patient's size, body surface area, age, the particular compound to be administered, sex, time and route of administration, general health, and other drugs being administered concurrently. Progress can be monitored by periodic assessment. A typical dose can be, for example, in the range of 1 µg to 1000 mg; however, doses below or above this exemplary range are envisioned, especially considering the aforementioned factors. Generally, the regimen as a regular administration of the pharmaceutical composition should be in the range of 1 µg to 100 mg units per day. If the regimen is a continuous infusion, it should also be in the range of 1 µg to 1 mg units per kilogram of body weight per minute, respectively. Preferably, the pharmaceutical composition is administered once to the subject, i.e., preferably, is used as a one-time treatment. Depending on the subject and the mode of administration, the quantity of substance administration may vary over a wide range to provide from about 0.01 mg per kg body mass to about 100 mg per kg body mass. The pharmaceutical compositions and formulations referred to herein are administered at least once in order to treat or ameliorate or prevent a disease or condition recited in this specification. However, the said pharmaceutical compositions may be administered more than one time, for example from two to 50 times, more preferably from five to 50 times. Administration may be continuous, e.g. as an infusion or by inhalation, hourly, two to 6 times daily, daily, every other day, every three days, weekly, biweekly, or monthly, preferably is continuous, hourly, two to 6 times daily, or daily. Preferably, administration is adjusted to maintain an effective concentration in the body of a subject for the time period intended. Progress can be monitored by periodic assessment. Preferably, the dosage of the compound is in accordance with the known indications of said compound; however, other dosages, in particular higher doses, may be envisaged.

Advantageously, it was found in the work underlying the present invention that the fibril peptides of the present invention form fibrils which associate with particular compounds such as viral particles and thereby strongly enhance uptake of such particles into host cells. Since the fibril peptides are derived from host proteins, i.e. from polypeptides to which the host, in particular a human, is tolerant, the fibril peptides are essentially non-immunogenic.

The definitions made above apply mutatis mutandis to the following. Additional definitions and explanations made further below also apply for all embodiments described in this specification mutatis mutandis.

The present invention further relates to a method of delivering a cargo comprised in a particle into a host cell, comprising
a) contacting said host cell with said particle and with a fibril peptide as specified herein; and, thereby
b) delivering said cargo into said host cell.

The method of the present invention, preferably, is an *in vitro* method. The method may, however, also be used *in vivo.* In case the method is performed on an experimental animal, the experimental animal is preferably sacrificed. Moreover, the method may comprise steps in addition to those explicitly mentioned above. For example, further steps may relate, e.g., to providing host cells for step a), or further uses of the cells produced in step b). Moreover, one or more of said steps may be performed or assisted by automated equipment.

The term "cargo", as used herein, relates in principle to any and all compounds or compositions of matter suitable for packaging into a particle as specified herein below and for which it is desirable to deliver them into a host cell. Thus, the cargo preferably is or comprises a chemical compound, preferably a pharmaceutical compound. More preferably, the cargo is a biological macromolecule, preferably a polynucleotide. Preferably, said polynucleotide is a viral genome or part thereof, more preferably is a genetically modified viral genome or part thereof. Thus, the cargo may comprise a transgene which shall be delivered to a target cell. As will be understood by the skilled person, the transgene may comprise any nucleic acid sequence deemed appropriate by the skilled person, such as an expression construct for a polypeptide and/or an RNA, a silencing construct, a Cas nuclease and/or gRNA expression constructs, one or more marker genes, a suicide gene, and the like. Preferably, the cargo is an expression construct for a chimeric antigen receptor (CAR); preferably, the host cell is a T cell in such case.

The term "particle", as used herein, relates to any particle of a suitable size to be taken up by a cell and comprising or adapted to take up at least one cargo. Preferably, the particle is an anionic particle, i.e. has a negative net surface charge, under standard conditions. Preferably, the particle has a size of from 1 nm to 1 µm, preferably of from 10 nm to 500 nm, more preferably of from 25 nm to 400 nm, still more preferably of from 50 nm to 300 nm, most preferably of from 100 nm to 200 nm, wherein, preferably, the size is determined as the average diameter. Preferably, the particle has an outer layer comprising or consisting of at least one polypeptide; thus, the particle may e.g. a non-enveloped virus or an enveloped virus, or a virus-like particle (VLP). More preferably, the particle has an outer layer comprising a lipid bilayer; thus, preferably, the particle is a liposome, e.g. a membrane vesicle or an artificial liposome. Preferably, the lipid bilayer of the particle comprises at least one fusogenic polypeptide adapted for mediating fusion of the lipid bilayer with a cell membrane. Preferably, the aforesaid lipid bilayer is derived from a host cell; thus, preferably, the particle is an enveloped virus or a VLP thereof, preferably a retrovirus, more preferably a lentivirus, most preferably a human immunodeficiency virus (HIV) or a VLP thereof. Also preferably, the particle is selected from the list consisting of an enveloped virus, a retrovirus, a retroviral particle, a pseudotyped retroviral particle, a lentivirus, a lentiviral particle, a pseudotyped lentiviral particle, a VSV-G glycoprotein pseudotyped retro- and/or lentiviral particle, a GaLV glycoprotein pseudotyped retro- and/or lentiviral particle, a RD114 glycoprotein pseudotyped retro- and/or lentiviral particle, a MLV glycoprotein pseudotyped, retro- and/or lentiviral particle, and a spumavirus. As is understood by the skilled person, it may not be necessary that the components of the particle reach the interior of the cell, i.e. the cytoplasm, the nucleus, and/or another cell compartment; preferably, it may not even be necessary that any component of the particle except the cargo reaches the interior of the cell. Also, it may be that the cargo is delivered only partially, e.g. only a part of a fusion polypeptide may be delivered; also, of a given number of particles, only a fraction may deliver their cargo into a cell. In particular in case the particle is a virus or VLP and/or the cargo is a polynucleotide, only 0.1% of said particles or even less may deliver their cargo.

As used herein, the term "host cell" relates to any cell preferably capable of receiving a cargo via a particle as specified herein above. More preferably, the host cell is capable of receiving and, preferably expressing; a cargo polynucleotide. Preferably, the host cell is a eukaryotic cell, preferably a yeast cell, e.g. a cell of a strain of baker's yeast, or is an animal cell. More preferably, the host cell is an insect cell or a mammalian cell, in particular a mouse or rat cell. Most preferably, the host cell is a human cell. Preferably, the host cell is an immune cell, preferably a T-cell, a cancer cell, a primary cell, a B cell, a B/T cell hybrid, a hematopoietic progenitor cell, a peripheral blood lymphocyte, a stem cell-like cell, a macrophages, a CD34+ cell, or a cell line, preferably selected from the list consisting of human 293T , HeLa , CEM-M7, TZM-bl, K562, HFF, and NIH3T3 cells.

The present invention further relates to a fibril peptide of the invention for use in the treatment of disease.

The present invention also relates to a fibril peptide of the invention for use in the treatment and/or prevention of cancer, genetic disease, and/or neurodegenerative disease.

The terms "treating" and "treatment" refer to an amelioration of a disease or disorder referred to herein or the symptoms accompanied therewith to a significant extent; as used herein, the term includes prevention of deterioration of a disease, disorder, or symptoms associated therewith. Said treating as used herein also includes an entire restoration of health with respect to the diseases or disorders referred to herein. It is to be understood that treating, as the term is used herein, may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 10%, at least 20% at least 50% at least 60%, at least 70%, at least 80%, or at least 90% of the subjects of a given cohort or population.

The term "preventing" refers to retaining health with respect to the diseases or disorders referred to herein for a certain period of time in a subject. It will be understood that said period of time may be dependent on the amount of the drug compound which has been administered and individual factors of the subject discussed elsewhere in this specification. It is to be also understood that prevention may not be effective in all subjects treated with the compound according to the present invention. However, the term requires that, preferably, a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well-known statistic evaluation tools discussed elsewhere in this specification.

The term "cancer", as used herein, relates to a disease of a subject, characterized by uncontrolled growth by a group of body cells ("cancer cells"). This uncontrolled growth may lead to tumor formation and may be accompanied by intrusion into and destruction of surrounding tissue (invasion) and possibly spread of cancer cells to other locations in the body (metastasis). Preferably, also included by the term cancer is a relapse. Thus, preferably, the cancer is a non-solid cancer, a solid cancer, a metastasis, and/or a relapse thereof. Preferably, the cancer is characterized by local or systemic immune suppression, i.e. preferably, the cancer is not recognized by the subject's immune system. Preferably, cancer treatment comprises immunotherapy, preferably cell-based immunotherapy. The term "immunotherapy", as used herein, relates to the treatment of cancer by modulation of the immune response of a subject. Said modulation may be inducing, enhancing, or suppressing said immune response, e.g. by administration of CAR T cells obtained with a fibril peptide and specifically recognizing cancer cells. The term "cell-based immunotherapy" relates to a cancer therapy comprising application of immune cells, e.g. T-cells, preferably tumor-specific NK cells, to a subject. Also preferably, cancer treatment further comprises at least one of surgery, chemotherapy, and radiotherapy.

The term "genetic disease", as used herein, relates to a disease causally linked to one or more modification(s), preferably mutation(s), in the genome of a subject. Thus, preferably, the genetic disease is causally linked to one or more epigenetic changes, more preferably is causally linked to one or more genetic mutations. As will be understood, symptoms of a genetic disease often are caused by expression of a mutated gene and/or lack of expression of a gene providing normal function of the gene product in one or more specific tissue(s) and/or cell type(s), and treatment may comprise provision of an unmutated copy of a disease-related gene to a host cell. Thus, it may be preferable to genetically modify only those cells in which the mutation contributes to disease. Preferably, the genetic disease is Duchenne muscular dystrophy, Huntington's disease, Hemophilia A/B, cystic fibrosis, myotubular myopathy, a glycogen storage disorder, or sickle cell anemia, the causes and symptoms of which are known to the skilled person from textbooks of medicine.

The term "neurodegenerative disease" relates to a disease caused by progressive loss of structure and/or function of neurons in the peripheral and/or central nervous system of an individual. Preferably, the neurodegenerative disease is a neurodegenerative disease of motoneurons and/or a neurodegenerative disease of the central nervous system. Preferably, the neurodegenerative disease is Alzheimer's disease, amyotrophic lateral sclerosis (ALS), Parkinson's disease, or a spinocerebellar ataxia, preferably spinocerebellar ataxia type 1 (SCA1). As will be understood, many neurodegenerative diseases are genetic diseases.

The present invention also relates to a fibril peptide of the invention for use in vaccination. Preferably, the fibril peptide is bound non-covalently or covalently to an antigen in such case. More preferably, the fibril peptide is comprised in a fusion polypeptide comprising the fibril peptide and an antigenic peptide or polypeptide in such case, wherein the antigenic peptide or polypeptide preferably is fused to the N-terminus and/or the C-terminus of the fibril peptide.

The term "vaccination", as used herein, preferably, relates to administering the compounds as specified herein to elicit an immune response. Thus, vaccination stimulates the immune system and establishes or improves immunity to infection, cancer, or any other disease associated with new production of a non-self antigen and/or neoantigen in a subject. Preferably, vaccination allows for establishing or improving immunity to infection. It is to be understood that the vaccine according to the present invention may comprise further components, e.g. excipients, adjuvants, and/or further antigens. The skilled person will understand that vaccination may not elicit a significant immune response in all subjects vaccinated. Also, it is to be understood that vaccination may not be effective to prevent infection in all subjects vaccinated. However, the term requires that a, preferably statistically significant, portion of subjects of a cohort or population are effectively vaccinated. Whether a portion is statistically significant can be determined without further ado by the person skilled in the art using various well-known statistic evaluation tools as specified herein above. Preferably, vaccination induces a humoral immune response in a subject, i.e., preferably induces the production of antibodies recognizing, preferably specifically recognizing, the antigen comprised in the vaccine. The term "specifically recognizing" is understood by the skilled person as the property of a binding agent, e.g. an antibody, to specifically bind to a particular species of molecule, while other molecules from the same chemical class of molecules, e.g. proteins, are not recognized or are recognized to a much lesser extent. Also preferably, vaccination induces a cellular immune response in a subject, i.e. activation and/or expansion of immune cells, preferably T cells.

The present invention also relates to a use, preferably *in vitro* use, of a fibril peptide according to the present invention for delivering a cargo comprised in a particle into a cell.

Further, the present invention relates to a pharmaceutical composition comprising a fibril peptide according to the present invention.

The present invention further relates to a kit comprising the fibril peptide according to the present invention and at least one solvent and/or diluent.

The term "kit", as used herein, refers to a collection of the aforementioned compounds, compositions, means, or reagents which may or may not be packaged together. The components of the kit may be comprised by separate vials (i.e. as a kit of separate parts) or provided in a single vial. Moreover, it is to be understood that the kit of the present invention, preferably, is to be used for practicing the methods or uses referred to herein above. It is, preferably, envisaged that all components are provided in a ready-to-use manner for practicing the methods or uses referred to above. Further, the kit, preferably, contains instructions for carrying out said methods or uses. The instructions can be provided by a user's manual in paper or electronic form. In addition, the manual may comprise instructions for administration and/or dosage instructions using the kit of the present invention. Preferably, the kit comprises a particle as specified herein above, or means for production of a particle. Also preferably, the kit further comprises a cargo.

Preferably, the kit comprises a diluent and/or a means of administration. Appropriate diluents are described herein above; means of administration are all means suitable for administering the compound to a subject, a cell, or a tissue. Preferred means of administration are those known to the skilled person for the respective compound. The means of administration may include a delivery unit for the administration of the compound or composition and a storage unit for storing said compound or composition until administration. However, it is also contemplated that the means of the current invention may appear as separate devices in such an embodiment and are, preferably, packaged together in said kit. Preferred means for administration are those which can be applied without the particular knowledge of a specialized technician. In a preferred embodiment, the means for administration is a syringe, more preferably with a needle, comprising the compound or composition of the invention. In another preferred embodiment, the means for administration is an intravenous infusion (IV) equipment comprising the compound or composition. In still another preferred embodiment the means for administration is an inhaler comprising the fibril peptide of the present invention, wherein, more preferably, said fibril compound is formulated for administration as an aerosol.

The present invention also relates to a device comprising the fibril peptide according to the present invention.

The term "device", as used herein, relates to a system of means comprising at least the means operatively linked to each other as to allow administration of the fibril peptide, in particular the medicament, of the present invention. Thus, the device preferably is adapted for administration of the compound or composition. Preferred means for administering a compound or composition depend on the mode of administration envisaged and are well known in the art. How to link the means in an operating manner will depend on the type of means included into the device. Preferably, the means are comprised by a single device. The device may include a delivery unit for the administration of the fibril peptide and a storage unit for storing said fibril peptide until administration. However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician. Preferably, the device is a syringe, more preferably with a needle, comprising the compound or composition of the invention. Also preferably, the device is an intravenous infusion (IV) equipment comprising the compound or composition. Further preferably, the device is an endoscopic device comprising the compound or composition for flushing the lung or parts thereof or a body cavity. Preferably, the device is a device for *in vitro* administration of the fibril peptide to a host cell, more preferably a pipetting device or a transfection device, in particular a high-throughput cell handling device.

In view of the above, the following embodiments are particularly envisaged:
Embodiment 1: A fibril peptide consisting of a sequence of from 8 to 20 amino acids and wherein said fibril peptide comprises the amino acid sequence RTIFIISM or a sequence at least 75% identical thereto.
Embodiment 2: The fibril peptide of embodiment 1, wherein said fibril peptide comprises the amino acid sequence RTIFIISM (SEQ ID NO: 1) or a sequence at least 85% identical thereto.
Embodiment 3: The fibril peptide of embodiment 1 or 2, wherein said fibril peptide has a length of from 8 to 12 amino acids, preferably of from 8 to 10 amino acids.
Embodiment 4: The fibril peptide of any one of embodiments 1 to 3, wherein said fibril peptide comprises the amino acid sequence X¹TIFIISM (SEQ ID NO:2), RX²IFIISM (SEQ ID NO:3), RTX²FIISM (SEQ ID NO:4), RTIX²IISM (SEQ ID NO:5), RTIFX²ISM (SEQ ID NO:6), RTIFIX²SM SEQ ID NO:7), RTIFIIX²M (SEQ ID NO:8), or RTIFIISX² (SEQ ID NO:9), wherein X¹ is a positively charged amino acid, preferably R, K or H, and wherein X² is any amino acid.
Embodiment 5: The fibril peptide of any one of embodiments 1 to 4, wherein said fibril peptide comprises the amino acid sequence X¹TIFIISM (SEQ ID NO:2), RX²IFIISM (SEQ ID NO:3), RTX²FIISM (SEQ ID NO:4), RTIX²IISM (SEQ ID NO:5), RTIFX²ISM (SEQ ID NO:6), RTIFIX²SM SEQ ID NO:7), RTIFIIX²M (SEQ ID NO:8), or RTIFIISX² (SEQ ID NO:9), wherein X¹ is R or K and wherein X is selected from the list consisting of A, V, L, I, M, F, T, W, G, S, T, N, Q, R, H, K, and derivatives thereof.
Embodiment 6: The fibril peptide of any one of embodiments 1 to 5, wherein said fibril peptide comprises the amino acid sequence X¹TIFIISM (SEQ ID NO:2), RX²IFIISM (SEQ ID NO:3), RTX²FIISM (SEQ ID NO:4), RTIX²IISM (SEQ ID NO:5), RTIFX²ISM (SEQ ID NO:6), RTIFIX²SM SEQ ID NO:7), RTIFIIX²M (SEQ ID NO:8), or RTIFIISX² (SEQ ID N0:9), wherein X¹ is R or K and wherein X² is selected from the list consisting of A, V, L, I, M, F, T, W, G, S, T, N, Q, R, H, K and non-negatively charged derivatives thereof.
Embodiment 7: The fibril peptide of any one of embodiments 1 to 6, wherein said fibril peptide comprises the amino acid sequence X¹TIFIISM (SEQ ID NO:2), RX²IFIISM (SEQ ID NO:3), RTX²FIISM (SEQ ID NO:4), RTIX²IISM (SEQ ID NO:5), RTIFX²ISM (SEQ ID NO:6), RTIFIX²SM SEQ ID NO:7), RTIFIIX²M (SEQ ID NO:8), or RTIFIISX² (SEQ ID NO:9), wherein X¹ is R or K and wherein X² is selected from the list consisting of A, V, L, I, M, F, T, and and non-negatively charged derivatives thereof.
Embodiment 8: The fibril peptide of any one of embodiments 1 to 7, wherein said fibril peptide comprises the amino acid sequence X¹TIFIISM (SEQ ID NO:2), RX²IFIISM (SEQ ID NO:3), RTX²FIISM (SEQ ID NO:4), RTIX²IISM (SEQ ID NO:5), RTIFX²ISM (SEQ ID NO:6), RTIFIX²SM SEQ ID NO:7), RTIFIIX²M (SEQ ID NO:8), or RTIFIISX² (SEQ ID NO:9).
Embodiment 9: The fibril peptide of any one of embodiments 1 to 8, wherein said fibril peptide consists of the amino acid sequence RTIFIISMX³X⁴ (SEQ ID NO:10), RAIFIISMX³X⁴ (SEQ ID NO: 11), RTAFIISMX³X⁴ (SEQ ID NO: 12), RTIAIISMX³X⁴(SEQ ID NO:13), RTIFAISMX³X⁴ (SEQ ID NO:14), RTIFIASMX³X⁴ (SEQ ID NO:15), RTIFIIAMX³X⁴ (SEQ ID NO: 16), RTIFIISAX³X⁴ (SEQ ID NO: 17), wherein X³ and X⁴ are independently selected from any amino acid or wherein X³ and/or X⁴ are absent.
Embodiment 10: The fibril peptide of any one of embodiments 1 to 9, wherein X³ is selected from the list consisting of Y, A, V, L, I, M, F, T, W, G, S, T, N, Q, R, H, K, and derivatives thereof, preferably non-negatively charged derivatives thereof, more preferably wherein X³ is Y.
Embodiment 11: The fibril peptide of any one of embodiments 1 to 10, wherein X⁴ is selected from the list consisting of K, R, H, A, V, L, I, M, F, T, W, G, S, T, N, Q, and derivatives thereof, preferably non-negatively charged derivatives thereof, more preferably, wherein X⁴ is K.
Embodiment 12: The fibril peptide of any one of embodiments 6 to 11, wherein said derivative is a straight-chain amino acid, preferably homoalanine, norvaline, or norleucine; a D-amino acid, preferably D-alanine, D-leucine, or D-isoleucine; a non-alpha amino acid, preferably beta-alanine or gamma-amino-butyric acid; a derivative comprising an exchange of an atom by an atom from the same chemical group, preferably selenomethionine; and/or a naturally occurring derivative of an amino acid, preferably hypusine.
Embodiment 13: The fibril peptide of any one of embodiments 1 to 12, wherein said fibril peptide consists of the amino acid sequence RTIFIISM (SEQ ID NO:1), RAIFIISM (SEQ ID NO:18), RTAFIISM (SEQ ID NO:19), RTIAIISM (SEQ ID NO:20), RTIFAISM (SEQ ID NO:21), RTIFIASM (SEQ ID NO:22), RTIFIIAM (SEQ ID NO:23), RTIFIISA (SEQ ID NO:24), RTIFIISMY (SEQ ID NO:25), RAIFIISMY (SEQ ID NO:26), RTAFIISMY (SEQ ID NO:27), RTIAIISMY (SEQ ID NO:28), RTIFAISMY (SEQ ID NO:29), RTIFIASMY (SEQ ID NO:30), RTIFIIAMY (SEQ ID NO:31), RTIFIISAY (SEQ ID NO:32), RTIFIISMA (SEQ ID NO:33), RTIFIISMYK (SEQ ID NO:34), RAIFIISMYK (SEQ ID NO:35), RTAFIISMYK (SEQ ID NO:36), RTIAIISMYK (SEQ ID NO:37), RTIFAISMYK (SEQ ID NO:38), RTIFIASMYK (SEQ ID NO:39), RTIFIIAMYK (SEQ ID NO:40), RTIFIISAYK (SEQ ID NO:41), RTIFIISMAK (SEQ ID NO:42), or RTIFIISMYA (SEQ ID NO:43).
Embodiment 14: The fibril peptide of any one of embodiments 1 to 13, wherein said fibril peptide consists of the amino acid sequence RTIFIISM (SEQ ID NO: 1), RAIFIISM (SEQ ID NO:18), RTAFIISM (SEQ ID NO:19), RTIAIISM (SEQ ID NO:20), RTIFAISM (SEQ ID NO:21), RTIFIASM (SEQ ID NO:22), RTIFIIAM (SEQ ID NO:23), RTIFIISA (SEQ ID NO:24), or RTIFIISMYK (SEQ ID NO:34), preferably RTIFIISM (SEQ ID NO:1) or RTIFIISMYK (SEQ ID NO:34).
Embodiment 15: The fibril peptide of any one of embodiments 1 to 14, wherein all amino acids comprised in the fibril peptide are amino acids are L-amino acids.
Embodiment 16: The fibril peptide of any one of embodiments 1 to 15, wherein all amino acids comprised in the fibril peptide are alpha-amino acids.
Embodiment 17: The fibril peptide of any one of embodiments 1 to 16, wherein all amino acids comprised in the fibril peptide are proteinogenic amino acids.
Embodiment 18: The fibril peptide of any one of embodiments 1 to 17, wherein said fibril peptide has low immunogenicity, preferably is non-immunogenic to a mammal, preferably to a human.
Embodiment 19: A method of delivering a cargo comprised in a particle into a host cell, comprising
   a) contacting said host cell with said particle and with a fibril peptide according to any one of embodiments 1 to 19; and, thereby
   b) delivering said cargo into said host cell.
Embodiment 20: The method of embodiment 19, wherein said particle is a virus particle, a virus-like particle, or a liposome comprising at least one polypeptide of a virus.
Embodiment 21: The method of embodiment 19 or 20, wherein said virus is a retrovirus, preferably a human immunodeficiency virus.
Embodiment 22: The method of any one of embodiments 19 to 21, wherein said cargo comprised in said particle is a biological macromolecule or a pharmaceutical compound.
Embodiment 23: The method of any one of embodiments 19 to 22, wherein said cargo comprised in said particle is a polynucleotide, preferably a viral genome or part thereof, more preferably a genetically modified viral genome or part thereof.
Embodiment 24: The method of any one of embodiments 19 to 23, wherein said host cell is a mammalian cell, preferably a human cell.
Embodiment 25: The method of any one of embodiments 19 to 24, wherein said host cell is a T cell, a cancer cell, a primary cell, a B cell, a B/T cell hybrid, a hematopoietic progenitor cell, a peripheral blood lymphocyte, a stem cell-like cell, a macrophages, a CD34+ cell, or a cell line, preferably selected from the list consisting of human 293T cells, HeLa cells, CEM-M7, TZM-bl, K562, HFF, and NIH3T3.
Embodiment 26: The method of any one of embodiments 19 to 25, wherein said method is an in vitro method.
Embodiment 27: A fibril peptide according to any one of embodiments 1 to 18 for use in the treatment of disease.
Embodiment 28: A fibril peptide according to any one of embodiments 1 to 18 for use in the treatment and/or prevention of cancer, genetic disease, or neurodegenerative disease.
Embodiment 29: Use, preferably in vitro use, of a fibril peptide according to any one of embodiments 1 to 18 for delivering a cargo comprised in a particle into a cell.
Embodiment 30: A pharmaceutical composition comprising a fibril peptide according to any one of embodiments 1 to 18.
Embodiment 31: A kit comprising the fibril peptide according to any one of embodiments 1 to 18 and at least one solvent and/or diluent.
Embodiment 32: A device comprising the fibril peptide according to any one of embodiments 1 to 18.

All references cited in this specification are herewith incorporated by reference with respect to their entire disclosure content and the disclosure content specifically mentioned in this specification.

### Figure Legends

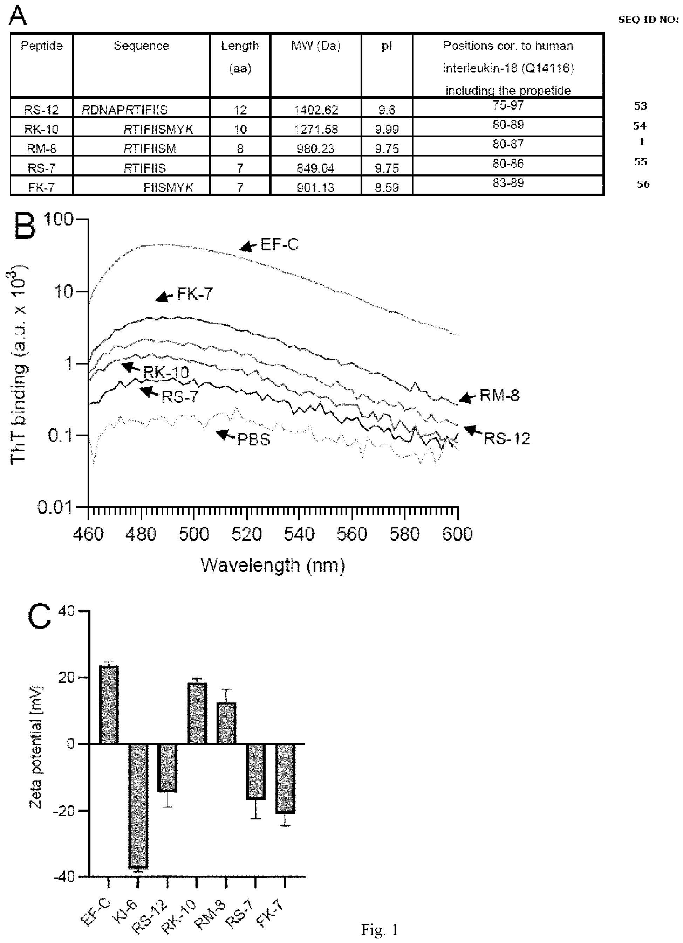

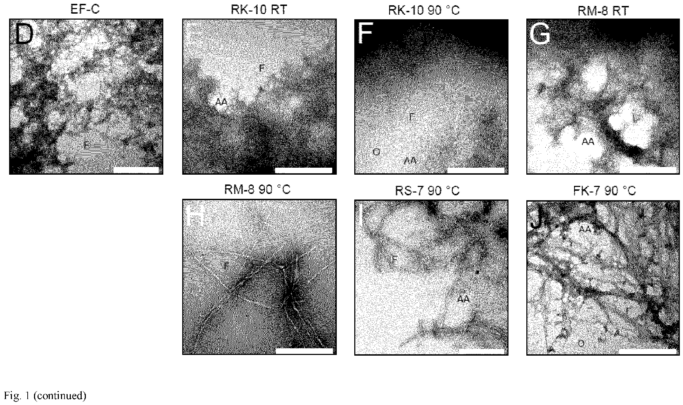

Fig. 1: Human interleukin-18 (IL-18) derived peptides form nanofibrils. (A) Table displaying sequences, physical and chemical parameters of peptides derived from IL-18. The positively charged amino acids are written in italics. (B) ThT fluorescence of the tested peptides and EF-C (Protransduzin^{®}-A) which were incubated at RT for 10 min. (C) Zeta potential of peptides which were incubated at RT for 10 min. Shown are average values (± SD) of duplicates from two independent experiments. (D-J) Transmission electron microscopy images of EF-C (D), RK-10 (E-F), RM-8 (G-H), RS-7 (I), and FK-7 (J). The peptides were incubated at RT for 10 min (D, E, G) or 90 °C for 30 min with subsequent cooling down overnight (F, H-J). Scale bars indicate 500 nm. A.u., arbitrary units; AA, amorphous aggregates; F, fibrils; MW, molecular weight; O, oligomers; RT, room temperature; SD, standard deviation; ThT, Thioflavin T.

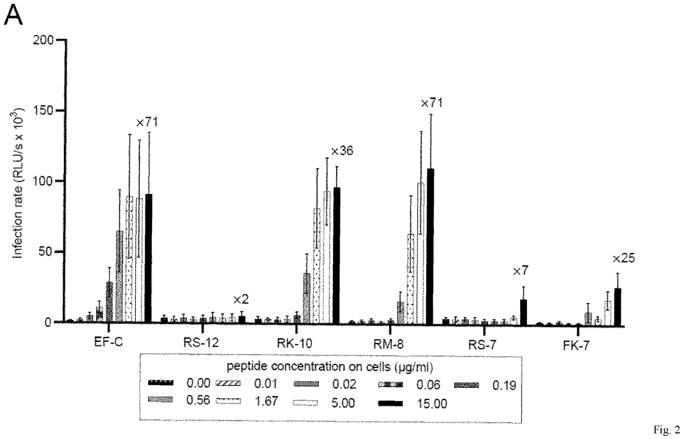

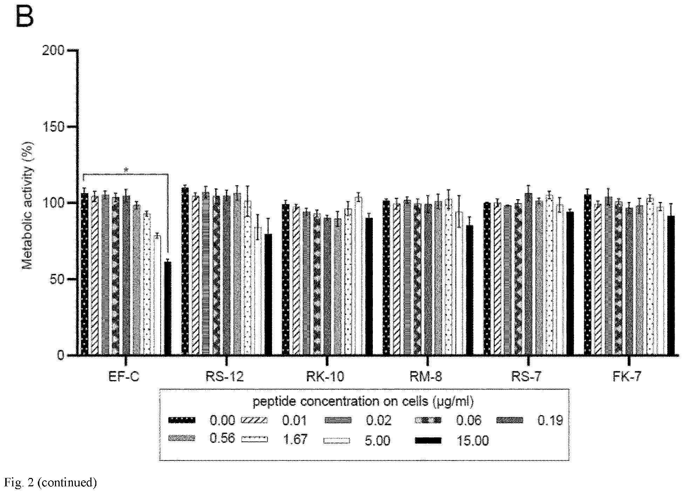

Fig. 2: Nanofibrils formed by IL-18 peptides enhance HIV-1 infection of TZM-bl cells without showing any cytotoxicity. (A) R5-tropic HIV-1 was treated with the peptides and mixtures were used to infect TZM-bl cells. Tat-inducible β-galactosidase activity was measured three days later. The numbers above the bars give the n-fold enhancement of infection relative to the control without peptide. The absolute values were slightly different in the control, but the decisive factor is the n-fold enhancement. Shown are average values (± SEM) of triplicate measurements from three independent experiments. (B) TZM-bl cells were treated with different peptide concentrations. MTT assay was performed three days later. The metabolic activity in % was calculated by dividing the averages of every peptide concentration by the values of cells in the absence of peptides. Shown are average values (± SD) of triplicate measurements from three independent experiments. Statistical analyses were performed using one-way ANOVA including the multiple comparison test Dunnett. Every concentration was compared with the control (cells without peptide). The concentrations not marked with an asterisk are not significantly different from the control. *p ≤ 0.05; RLU/s, relative light units per second; SD, standard deviation; SEM, standard error of the mean.

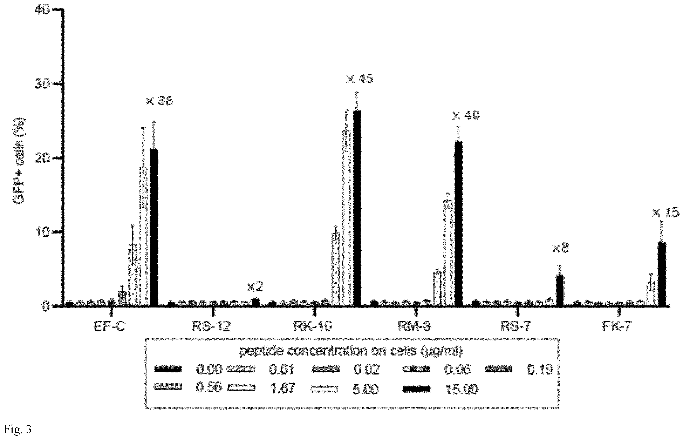

Fig. 3: Nanofibrils formed by IL-18 peptides enhance GALV-γ-RV infection of Jurkat cells. GALV-γ-RV was treated with the peptide and mixtures were used to infect Jurkat cells. GFP positive (GFP+) cells were determined two days later by flow cytometry. The numbers above the bars give the n-fold enhancement of infection relative to the average of controls without peptide. The absolute values were slightly different in the control, but the decisive factor is the n-fold enhancement. Shown are average values (± SD) of three independent experiments. GFP, green fluorescent protein. SD; standard deviation.

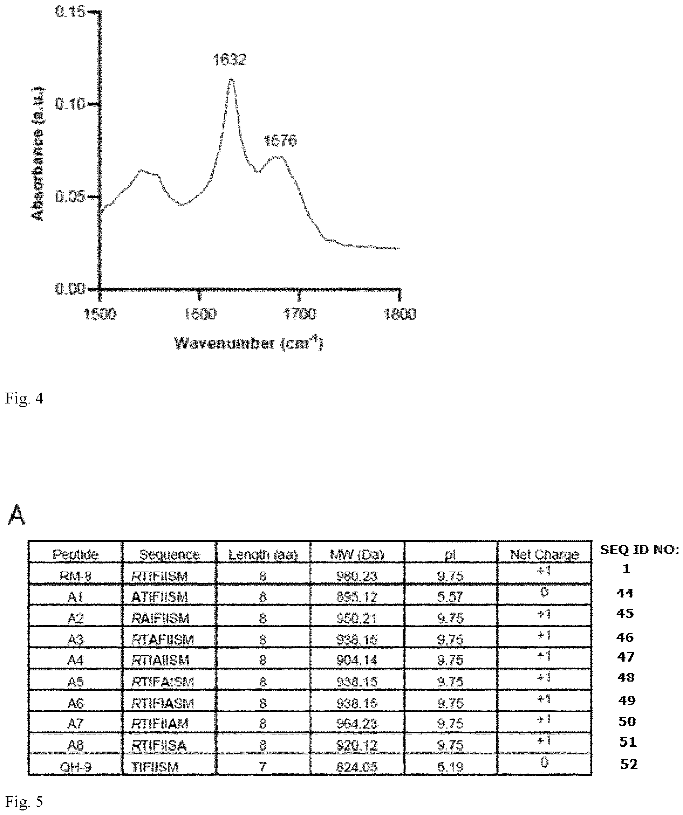

Fig. 4: FTIR spectrum of RM-8. The values in the plot refer to spectral maxima in the amide I region. RM-8 was incubated for 10 min at RT. A.u., arbitrary units.

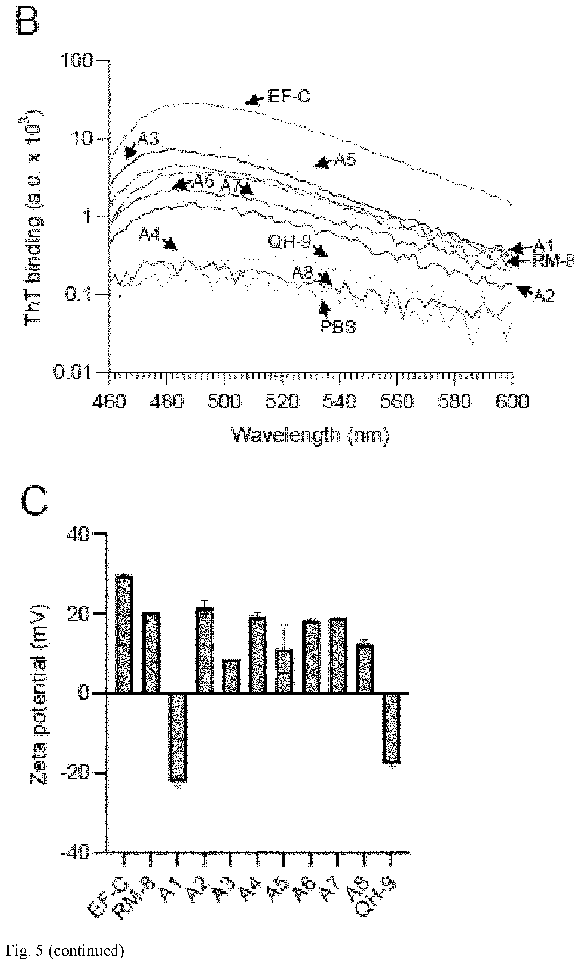

Fig. 5: RM-8 analogs from alanine scanning form positively charged nanofibrils. (A) Table displaying sequences, physical and chemical parameters of RM-8 and its analogs from alanine scanning (A1-8) and QH-9. The positively charged amino acids are written in italics. Residues mutated to alanine (A) are written in bold. (B) ThT fluorescence of alanine scanning derived peptides, RM-8, QH-9 and EF-C. (C) Zeta potential of peptides. Shown are average values (± SD) of duplicates. The peptides were incubated at RT for 10 min. A.u., arbitrary units. SD, standard deviation.

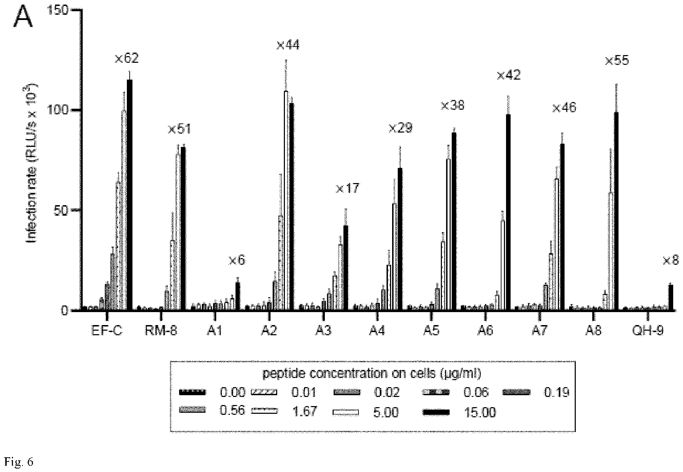

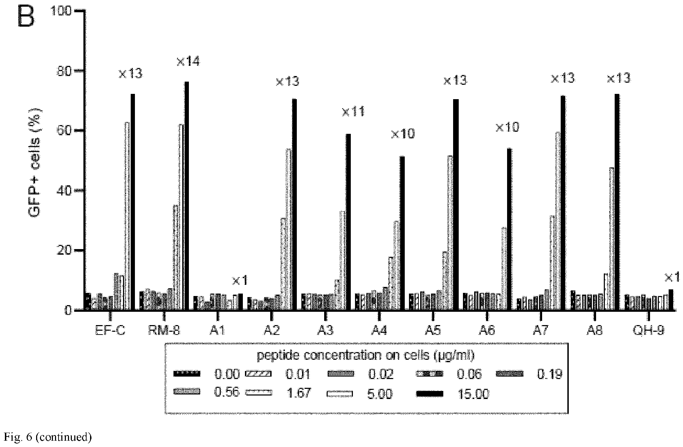

Fig. 6: RM-8 analogs from alanine scanning enhance HIV-1 and GALV-γ-RV infection. (A) R5-tropic HIV-1 was treated with peptides and mixtures were added to TZM-bl cells. Tat-inducible β-galactosidase activity was measured three days later. Shown are average values (± SD) of triplicate measurements. (B) GALV-γ-RV was treated with the peptide and mixtures were used to infect Jurkat cells. GFP positive (GFP+) cells were determined two days later by flow cytometry. The numbers above the bars give the n-fold enhancement of infection relative to the average of controls without peptide. The absolute values were slightly different in the control, but the decisive factor is the n-fold enhancement. GFP, green fluorescent protein; RLU/s, relative light units per second; SD, standard deviation.

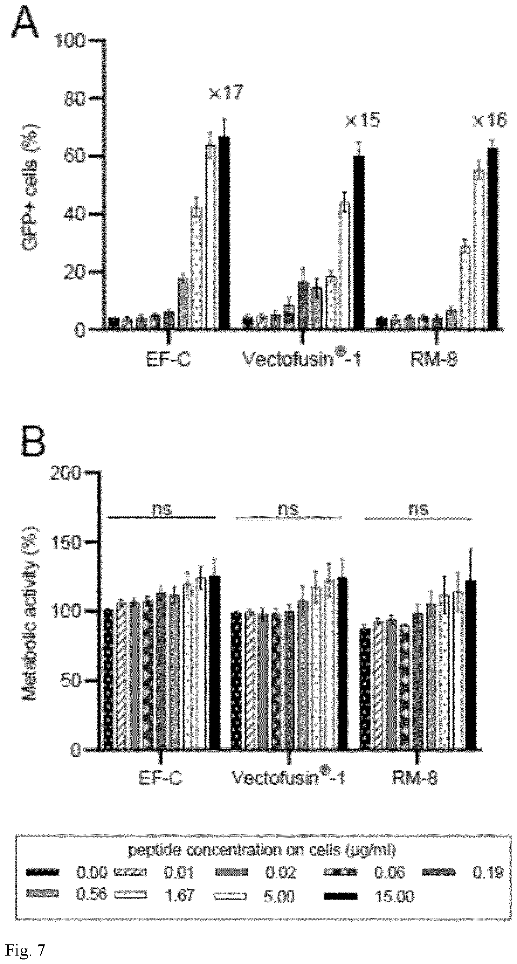

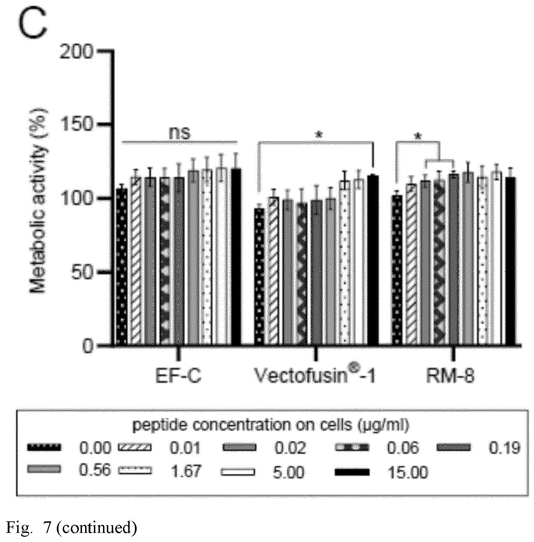

Fig. 7: Comparison of enhancing activity and cytotoxicity of RM-8 with the transduction enhancer Vectofusin^{®}-1. (A) GALV-γ-RV was treated with the peptide and mixtures were used to infect Jurkat cells. GFP positive (GFP+) cells were determined two days later by flow cytometry. The numbers above the bars give the n-fold enhancement of infection relative to the average of controls without peptide. Shown are average values (± SD) of three independent experiments. (B) MTT assay. (C) CellTiter-Glo^{®} Luminescent Cell Viability Assay. Both assays were performed two days after the peptide treatment of cells. The metabolic activity in % was calculated by dividing the averages of every peptide concentration by the values of cells in the absence of peptides. Shown are average values (± SD) of triplicate measurements from three independent experiments. Statistical analyses were performed using one-way ANOVA including the multiple comparison test Dunnett. Every concentration was compared with the control (cells without peptide). GFP, green fluorescent protein; Ns, not significant; *p ≤ 0.05; SD, standard deviation.

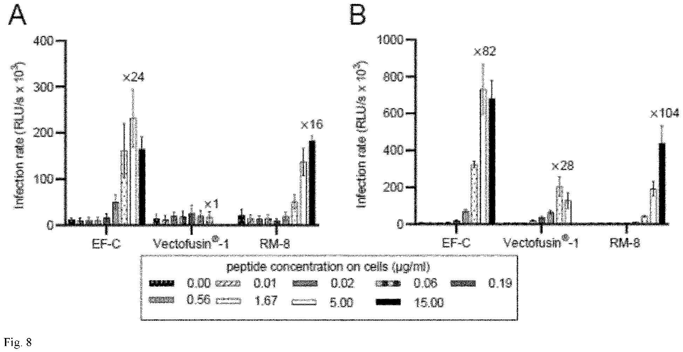

Fig. 8: RM-8 fibrils increase lentiviral transduction of HEK293T cells with higher efficiency than Vectofusin^{®}-1. (A) Lentiviral vectors pseudotyped with the glycoprotein of VSV were treated with the peptides and mixtures were used to infect HEK293T cells. The infection rates were determined two days later (B) Lentiviral vectors pseudotyped with a modified RD114/TR envelope glycoprotein were treated with the peptides and mixtures were used to infect HEK293T cells. The infection rates were determined three days later. The numbers above the bars give the n-fold enhancement of infection relative to the average of controls without peptide. Shown are average values (± SD) of three independent experiments. SD, standard deviation.

The following Examples shall merely illustrate the invention. They shall not be construed, whatsoever, to limit the scope of the invention.

### Material and Methods

HIV-1 was produced from pBRNL4.39-92TH14, a plasmid containing a CCR5 tropic molecular HIV-1 clone (Papkalla et al., 2002). Infectious HIV-1 stocks were prepared by transfection of 293T cells by calcium phosphate transfection and HIV-1 infection of TZM-bl cells was determined by detecting cellular β-galactosidase activity.
Retroviral vetors (RVs) were produced by cotransfection of a GFP expressing Mouse leukaemia virus (MLV) vector with E848 pCsGPpA-ed and glycoprotein derived from Gibbon ape Leukemia Virus (GALV). GFP positive cells were determined by flow cytometry. Lentiviral vectors (LVs) pseudotyped with the glycoprotein of Vesicular Stomatitis Virus (VSV-G) were generated by cotransfection of 293T cells with pSEW-luc2 and d8.9 and glycoprotein derived from VSV.
LV was generated by cotransfection of 293T cells with an env deleted pBRHIV-1 NL4 3 derivative encoding luciferase instead of nef and phCMV-RD114/TR encoding chimeric envelope glycoproteins derived from Feline Leukemia Virus (RD114) with the cytoplasmic tail derived from the MLV gp.

The peptide was synthesized by KE Biochem Co., Ltd (Shanghai City, China) and purity was proved by Core Facility Functional Peptidomics (Ulm University). The peptide was solved in HFIP (1,1,1,3,3,3-Hexafluoro-2-propanol) and were sonicated using an Ultrasonic cleaner. The peptides were stored at -20 °C and for each experiment they were freshly dissolved in DMSO to 10 mg/ml; to generate fibrils, the peptides were diluted in PBS to get 2 mg/ml or 1 mg/ml and incubated for 10 min at RT.

### Results

### Interleukin-18 derived peptides form positively charged nanofibrils.

Peptides derived from human interleukin-18 were identified by an in silico-screening approach where the human protein interleukin-18 (Q14116) was analyzed by the amyloid prediction tools ZipperDB, TANGO 2.0 and PASTA (Fig. 1A). To examine if these endogenous peptides form nanofibrils, the freshly prepared peptides were incubated with the amyloid specific dye Thioflavin T (ThT), which fluorescence increases upon binding to amyloid fibrils. ThT fluorescence scans showed that the IL-18 derived peptides which were freshly prepared by diluting the DMSO stock (10 mg/ml) fivefold in PBS (2 mg/ml) and incubation at room temperature (RT) for 10 min resulted in a clear ThT signal (Fig. 1B). Further evidence that the IL-18 peptides form nanofibrils was derived by transmission electron microscopy (TEM) analysis which showed a fibrillar structure similar to the positive control EF-C (Fig. 1D-J). To determine if the IL-18 peptides have the ability to form positively charged fibrils the zeta potential was measured. Only RK-10 and RM-8 nanofibrils displayed a positive zeta potential such as EF-C (Fig. 1C).

### Nanofibrils formed by IL-18 peptides enhance HIV-1 infection without showing cytotoxicity

To determine the effect on HIV-1 infection, stocks of the CCR5 tropic HIV-1 were generated by transient transduction of 293T cells with proviral DNA and harvested 2 days later. The HIV-1 stock was titrated and a dilution factor of 125 was chosen for infection of TZM-bl cells to reach a maximal infection enhancement. HIV-1 virus was added to serially diluted IL-18 peptides and mixtures were used to infect TZM-bl cells which contain the reporter gene *β-galactosidase* under the control of the HIV-1-LTR promoter (Wei et al., 2002). Tat-inducible β-galactosidase activity was measured 3 days later showing a dose-dependably enhancement of HIV-infection only for RK-10, RM-8 and the positive control EF-C (Fig. 2A). For example, treatment of the virus with 150 µg/ml of RK-10, RM-8 and EF-C enhanced HIV-1 infection between 36 to 71-fold, whereas RS-12, RS-7 and FK-7 displayed weak or no effects. To analyze possible cytotoxic effects, freshly dissolved peptides were incubated with TZM-bl cells for three days and then the metabolic activity was assessed by measurement of the conversion of yellow MTT to purple formazan crystals by metabolic active cells. The MTT assay demonstrated that none of the IL-18 peptides were cytotoxic (Fig. 2B).

### Nanofibrils formed by IL-18 peptides fibrils enhance GALV-γ-RV infection

To investigate if RM-8 fibrils also increase transduction efficiency of γ-retroviral vectors (γ-RVs) which are currently used for the production of CAR T cells (Scholler et al, 2012; Muul et al., 2003; Macpherson et al., 2005), a GFP encoding RV pseudotyped with the glycoprotein derived from Gibbon ape Leukemia Virus (GALV-γ-RV) was generated. Serially diluted peptides were incubated with GALV-γ-RV and Jurkat cells were infected with this fibril-mixture mix. Two days later the GFP-expression was determined by flow cytometry analysis. It was found that treatment with RM-8, RK-10 and EF-C fibrils enhanced gene delivery rates from around 1 % in the control to maximal 20-30 % GFP positive cells whereas the other peptides exhibited only a weak enhancing activity (Fig. 3).

### Structural characterization of the shortest peptide RM-8

The endogenous 8-mer peptide RM-8 was the shortest IL-18 peptide which showed enhancing activity for HIV-1 and GALV-γ-RV infection comparable to EF-C. To analyze the secondary structure of RM-8 fibrils an attenuated total reflection Fourier transform infrared (ATR-FTIR) spectroscopy measurement was performed according to Sieste et al., 2021. Therefore, RM-8 was freshly prepared by diluting the DMSO stock (10 mg/ml) tenfold in PBS (1 mg/ml) and incubated at RT for 10 min. The FTIR spectrum was dominated by amide I maxima at approximately 1632 and 1676 cm¹ (Fig. 4) which is characteristic for the presence of a high content of intermolecular β-sheet structures (Zandomeneghi et al., 2004). The calculation of native intramolecular secondary elements resulted in a β-sheet content of approximately 75.9 ± 3.4 %. Furthermore, the conversion rate (CR) of RM-8 which was incubated as described before was determined according to Schilling et al., 2019. The CR measures the amount of peptide monomers that form fibrils. RM-8 showed a high CR of 97.0 ± 2.6 % demonstrating a high fibrillation after 10 min.

### RM-8 analogs from alanine scanning form positively charged nanofibrils and enhance HIV-1 and GALV-γ-RV infection

To investigate the function of each amino acid in RM-8 concerning its ability to form fibrils and to enhance infection, an alanine scanning was performed. Therefore, each residue of RM-8 was mutated to alanine (Fig. 5A). Additional to the RM-8 analogs (A1-A8), the peptide QH-9 was chosen as a negative control. QH-9 is a variant of RM-8 that lacks the positive amino acid arginine at the N-terminus leading to a negative charge at physiological pH. To investigate if RM-8 analogs and QH-9 form nanofibrils, the reactivity of these peptides with ThT was analyzed. A ThT signal was detected for RM-8 analogs except for A4, A8 an QH-9 (Fig. 5B). Next, the zeta potential was determined. As expected, A1 and QH-9 displayed a negative zeta potential due to their pI < 7 while the other nanofibrils showed a positive zeta potential (Fig. 5C).
Next, the enhancing effect of RM-8 analogs on HIV-1 and GALV-γ-RV infection was examined. Therefore, HIV-1 infection assay was performed as described above. A concentration-dependent enhancement of HIV-1 infection was measured for cationic peptides from the alanine scanning (Fig. 6A). The anionic peptides A1 and QH-9 amplified the infection slightly at their highest concentration.
The concentration of GALV-γ-RV particles was increased by a factor of 10 compared to the previous experiments to achieve a higher yield of GFP positive cells. EF-C increased the number of GFP positive cells from 5.4 % to 72 % by a factor of 13 (Figure 6B). For RM-8 a 14-fold increase in infection could be observed, which led to approx. 76 % GFP positive cells. The fibrils formed by the cationic RM-8 analogs also increased gene transfer rates of GALV-γ-RV similar to RM-8. No clear infection enhancement was observed for the anionic peptides A1 and QH-9.

### RM-8 increases transduction efficiency of lentiviral and retroviral vectors pseudotyped with various glycoproteins

The effect of RM-8 on transduction enhancement was analyzed for transduction by LVs and RVs with different envelopes routinely used for genetic modifications including the glycoprotein derived from Gibbon ape Leukemia Virus (GALV), from Vesicular Stomatitis Virus (VSV-G) and a chimeric envelope glycoprotein (RD114/TR) derived from Feline Leukemia Virus (RD114) with the cytoplasmic tail derived from the MLV gp. Additionally, the enhancing activity of RM-8 for these viral particles was compared to those of Vectofusin^{®}-1, which is a fibril-forming transduction enhancer which was incorporated into the T Cell transduction process on the Clin-iMACS Prodigy^{®} of Miltenyi Biotec for automated production of T cells (Radek et al., 2019). Therefore, GFP encoding RV (Fig. 7A) and luciferase encoding LV (Fig. 8A, B) pseudotyped with different glycoproteins were combined with the peptides and target cells were infected with those mixtures. Flow cytometry analysis revealed a comparable infection enhancement of GALV-γ-RV for all three peptides in which the number of GFP positive cells was extended from about 5% to 60-69 % (Fig. 7A). To determine possible cytotoxic effects of the peptides, MTT assay and CellTiter-Glo^{®} Luminescent Cell Viability Assay were performed. CellTiter-Glo^{®} Luminescent Cell Viability Assay can be used to measure the viability of cells based on the quantification of adenosine triphosphate (ATP), which is an indicator of metabolically active cells. Both cell viability assays did not reveal any cytotoxic effects of the peptides on Jurkat cells (Fig. 7B, C). However, a significant increase in metabolic activity was observed for some concentrations (Fig. 7C). Luciferase assay of infected HEK293T cells performed two days later demonstrated that RM-8 and EF-C enhanced transduction rates of VSV-G pseudotyped LVs whereas Vectofusin^{®}-1 did not show any enhancing activity (Fig. 8A). Similar results were obtained for RD114/TR infection assay where RM-8 exhibited much higher gene delivery rates than Vectofusin^{®}-1 (Fig. 8B). These data obtained with different lentiviral and retroviral vectors carrying different glycoproteins show that RM-8 fibrils have a broad-based enhancing activity.

### Literature:

Cornetta et al., J. Virol. Methods 1989, 23 (2), 187
Macpherson et al., J. Gene Med. 2005, 7 (5), 552-564.
Majdoul et al., J. Biol. Chem. 2016, 291 (5), 2161
Munch et al., Cell 2007, 131 (6), 1059
Muul et al., Blood 2003, 101 (7), 2563-2569
Papkalla et al., J. Virol. 2002, 76 (16), 8455-8459
Radek et al., Hum. Gene Ther. 2019, 30 (12), 1477-1493.
Schilling et al., Adv. Funct. Mater. 2019, 29, 1809112
Sieste et al., Adv. Funct. Mater. 2021, 2009382
Scholler et al., Sci. Transl. Med., 2012, 4 (132), 132ra53
Wei et al., Antimicrob. Agents Chemother. 2002, 46 (6), 1896-1905.
Wurm et al., J. Gene Med. 2010, 12 (2), 137
Yolamanova et al., Nat. Nanotechnol. 2013, 8 (2), 130
Zandomeneghi et al., Protein Sci., 2009, 13, 3314-3321

SEQUENCE LISTING

<110>  Universität Ulm

<120>  Fibril Peptides

<130>  UU16229EP

<160>  56

<170>  PatentIn version 3.5

<210>  1
<211>  8
<212>  PRT
<213>  Homo sapiens

<400>  1

Arg Thr Ile Phe Ile Ile Ser Met
1               5


<210>  2
<211>  8
<212>  PRT
<213>  Artificial Sequence

<220>
<223>  fibril peptide 2


<220>
<221>  VARIANT
<222>  (1)..(1)
<223>  X is a positively charged amino acid

<400>  2

Xaa Thr Ile Phe Ile Ile Ser Met
1               5


<210>  3
<211>  8
<212>  PRT
<213>  Artificial Sequence

<220>
<223>  fibril peptide 3


<220>
<221>  VARIANT
<222>  (2)..(2)
<223>  X is any amino acid

<400>  3

Arg Xaa Ile Phe Ile Ile Ser Met
1               5

<210>  4
<211>  8
<212>  PRT
<213>  Artificial Sequence

<220>
<223>  fibril peptide 4


<220>
<221>  VARIANT
<222>  (3)..(3)
<223>  X is any amino acid

<400>  4

Arg Thr Xaa Phe Ile Ile Ser Met
1               5


<210>  5
<211>  8
<212>  PRT
<213>  Artificial Sequence

<220>
<223>  fibril peptide 5


<220>
<221>  VARIANT
<222>  (4)..(4)
<223>  X is any amino acid

<400>  5

Arg Thr Ile Xaa Ile Ile Ser Met
1               5


<210>  6
<211>  8
<212>  PRT
<213>  Artificial Sequence

<220>
<223>  fibril peptide 6


<220>
<221>  VARIANT
<222>  (5)..(5)
<223>  X is any amino acid

<400>  6

Arg Thr Ile Phe Xaa Ile Ser Met
1               5


<210>  7
<211>  8
<212>  PRT

<213>  Artificial Sequence

<220>
<223>  fibril peptide 7


<220>
<221>  VARIANT
<222>  (6)..(6)
<223>  X is any amino acid

<400>  7

Arg Thr Ile Phe Ile Xaa Ser Met
1               5


<210>  8
<211>  8
<212>  PRT
<213>  Artificial Sequence

<220>
<223>  fibril peptide 8


<220>
<221>  VARIANT
<222>  (7)..(7)
<223>  X is any amino acid

<400>  8

Arg Thr Ile Phe Ile Ile Xaa Met
1               5


<210>  9
<211>  8
<212>  PRT
<213>  Artificial Sequence

<220>
<223>  fibril peptide 9


<220>
<221>  VARIANT
<222>  (8)..(8)
<223>  X is any amino acid

<400>  9

Arg Thr Ile Phe Ile Ile Ser Xaa
1               5


<210>  10
<211>  10
<212>  PRT
<213>  Artificial Sequence

<220>

<223>  fibril peptide 10

<220>
<221>  VARIANT
<222>  (9)..(10)
<223>  X is any amino acid or absent

<400>  10

Arg Thr Ile Phe Ile Ile Ser Met Xaa Xaa
1               5                   10

<210>  11
<211>  10
<212>  PRT
<213>  Artificial Sequence

<220>
<223>  fibril peptide 11

<220>
<221>  VARIANT
<222>  (9)..(10)
<223>  X is any amino acid or absent

<400>  11

Arg Ala Ile Phe Ile Ile Ser Met Xaa Xaa
1               5                   10

<210>  12
<211>  10
<212>  PRT
<213>  Artificial Sequence

<220>
<223>  fibril peptide 12

<220>
<221>  VARIANT
<222>  (9)..(10)
<223>  X is any amino acid or absent

<400>  12

Arg Thr Ala Phe Ile Ile Ser Met Xaa Xaa
1               5                   10

<210>  13
<211>  10
<212>  PRT
<213>  Artificial Sequence

<220>
<223>  fibril peptide 13

<220>
<221>  VARIANT
<222>  (9)..(10)
<223>  X is any amino acid or absent

<400>  13

Arg Thr Ile Ala Ile Ile Ser Met Xaa Xaa
1               5                   10


<210>  14
<211>  10
<212>  PRT
<213>  Artificial Sequence

<220>
<223>  fibril peptide 14


<220>
<221>  VARIANT
<222>  (9)..(10)
<223>  X is any amino acid or absent

<400>  14

Arg Thr Ile Phe Ala Ile Ser Met Xaa Xaa
1               5                   10


<210>  15
<211>  10
<212>  PRT
<213>  Artificial Sequence

<220>
<223>  fibril peptide 15


<220>
<221>  VARIANT
<222>  (9)..(10)
<223>  X is any amino acid or absent

<400>  15

Arg Thr Ile Phe Ile Ala Ser Met Xaa Xaa
1               5                   10


<210>  16
<211>  10
<212>  PRT
<213>  Artificial Sequence

<220>
<223>  fibril peptide 16


<220>
<221>  VARIANT
<222>  (9)..(10)

<223>  X is any amino acid or absent

<400>  16

Arg Thr Ile Phe Ile Ile Ala Met Xaa Xaa
1               5                   10


<210>  17
<211>  10
<212>  PRT
<213>  Artificial Sequence

<220>
<223>  fibril peptide 17


<220>
<221>  VARIANT
<222>  (9)..(10)
<223>  X is any amino acid or absent

<400>  17

Arg Thr Ile Phe Ile Ile Ser Ala Xaa Xaa
1               5                   10


<210>  18
<211>  8
<212>  PRT
<213>  Artificial Sequence

<220>
<223>  fibril peptide 18

<400>  18

Arg Ala Ile Phe Ile Ile Ser Met
1               5


<210>  19
<211>  8
<212>  PRT
<213>  Artificial Sequence

<220>
<223>  fibril peptide 19

<400>  19

Arg Thr Ala Phe Ile Ile Ser Met
1               5


<210>  20
<211>  8
<212>  PRT
<213>  Artificial Sequence

<220>
<223>  fibril peptide 20

<400>  20

Arg Thr Ile Ala Ile Ile Ser Met
1               5


<210>  21
<211>  8
<212>  PRT
<213>  Artificial Sequence

<220>
<223>  fibril peptide 21

<400>  21

Arg Thr Ile Phe Ala Ile Ser Met
1               5


<210>  22
<211>  8
<212>  PRT
<213>  Artificial Sequence

<220>
<223>  fibril peptide 22

<400>  22

Arg Thr Ile Phe Ile Ala Ser Met
1               5


<210>  23
<211>  8
<212>  PRT
<213>  Artificial Sequence

<220>
<223>  fibril peptide 23

<400>  23

Arg Thr Ile Phe Ile Ile Ala Met
1               5


<210>  24
<211>  8
<212>  PRT
<213>  Artificial Sequence

<220>
<223>  fibril peptide 24

<400>  24

Arg Thr Ile Phe Ile Ile Ser Ala
1               5

<210>  25
<211>  9
<212>  PRT
<213>  Artificial Sequence

<220>
<223>  fibril peptide 25

<400>  25

Arg Thr Ile Phe Ile Ile Ser Met Tyr
1               5


<210>  26
<211>  9
<212>  PRT
<213>  Artificial Sequence

<220>
<223>  fibril peptide 26

<400>  26

Arg Ala Ile Phe Ile Ile Ser Met Tyr
1               5


<210>  27
<211>  9
<212>  PRT
<213>  Artificial Sequence

<220>
<223>  fibril peptide 27

<400>  27

Arg Thr Ala Phe Ile Ile Ser Met Tyr
1               5


<210>  28
<211>  9
<212>  PRT
<213>  Artificial Sequence

<220>
<223>  fibril peptide 28

<400>  28

Arg Thr Ile Ala Ile Ile Ser Met Tyr
1               5


<210>  29
<211>  9
<212>  PRT
<213>  Artificial Sequence

<220>
<223>  fibril peptide 29

<400>  29

Arg Thr Ile Phe Ala Ile Ser Met Tyr
1               5


<210>  30
<211>  9
<212>  PRT
<213>  Artificial Sequence

<220>
<223>  fibril peptide 30

<400>  30

Arg Thr Ile Phe Ile Ala Ser Met Tyr
1               5


<210>  31
<211>  9
<212>  PRT
<213>  Artificial Sequence

<220>
<223>  fibril peptide 31

<400>  31

Arg Thr Ile Phe Ile Ile Ala Met Tyr
1               5


<210>  32
<211>  9
<212>  PRT
<213>  Artificial Sequence

<220>
<223>  fibril peptide 32

<400>  32

Arg Thr Ile Phe Ile Ile Ser Ala Tyr
1               5


<210>  33
<211>  9
<212>  PRT
<213>  Artificial Sequence

<220>
<223>  fibril peptide 33

<400>  33

Arg Thr Ile Phe Ile Ile Ser Met Ala
1               5

<210>  34
<211>  10
<212>  PRT
<213>  Artificial Sequence

<220>
<223>  fibril peptide 34

<400>  34

Arg Thr Ile Phe Ile Ile Ser Met Tyr Lys
1               5                   10


<210>  35
<211>  10
<212>  PRT
<213>  Artificial Sequence

<220>
<223>  fibril peptide 35

<400>  35

Arg Ala Ile Phe Ile Ile Ser Met Tyr Lys
1               5                   10


<210>  36
<211>  10
<212>  PRT
<213>  Artificial Sequence

<220>
<223>  fibril peptide 36

<400>  36

Arg Thr Ala Phe Ile Ile Ser Met Tyr Lys
1               5                   10


<210>  37
<211>  10
<212>  PRT
<213>  Artificial Sequence

<220>
<223>  fibril peptide 37

<400>  37

Arg Thr Ile Ala Ile Ile Ser Met Tyr Lys
1               5                   10


<210>  38
<211>  10
<212>  PRT
<213>  Artificial Sequence

<220>
<223>  fibril peptide 38

<400>  38

Arg Thr Ile Phe Ala Ile Ser Met Tyr Lys
1               5                   10


<210>  39
<211>  10
<212>  PRT
<213>  Artificial Sequence

<220>
<223>  fibril peptide 39

<400>  39

Arg Thr Ile Phe Ile Ala Ser Met Tyr Lys
1               5                   10


<210>  40
<211>  10
<212>  PRT
<213>  Artificial Sequence

<220>
<223>  fibril peptide 40

<400>  40

Arg Thr Ile Phe Ile Ile Ala Met Tyr Lys
1               5                   10


<210>  41
<211>  10
<212>  PRT
<213>  Artificial Sequence

<220>
<223>  fibril peptide 41

<400>  41

Arg Thr Ile Phe Ile Ile Ser Ala Tyr Lys
1               5                   10


<210>  42
<211>  10
<212>  PRT
<213>  Artificial Sequence

<220>
<223>  fibril peptide 42

<400>  42

Arg Thr Ile Phe Ile Ile Ser Met Ala Lys
1               5                   10

<210>  43
<211>  10
<212>  PRT
<213>  Artificial Sequence

<220>
<223>  fibril peptide 43

<400>  43

Arg Thr Ile Phe Ile Ile Ser Met Tyr Ala
1               5                   10


<210>  44
<211>  8
<212>  PRT
<213>  Artificial Sequence

<220>
<223>  fibril peptide 44

<400>  44

Ala Thr Ile Phe Ile Ile Ser Met
1               5


<210>  45
<211>  8
<212>  PRT
<213>  Artificial Sequence

<220>
<223>  fibril peptide45

<400>  45

Arg Ala Ile Phe Ile Ile Ser Met
1               5


<210>  46
<211>  8
<212>  PRT
<213>  Artificial Sequence

<220>
<223>  fibril peptide 46

<400>  46

Arg Thr Ala Phe Ile Ile Ser Met
1               5


<210>  47
<211>  8
<212>  PRT
<213>  Artificial Sequence

<220>
<223>  fibril peptide 47

<400>  47

Arg Thr Ile Ala Ile Ile Ser Met
1               5


<210>  48
<211>  8
<212>  PRT
<213>  Artificial Sequence

<220>
<223>  fibril peptide 48

<400>  48

Arg Thr Ile Phe Ala Ile Ser Met
1               5


<210>  49
<211>  8
<212>  PRT
<213>  Artificial Sequence

<220>
<223>  fibril peptide 49

<400>  49

Arg Thr Ile Phe Ile Ala Ser Met
1               5


<210>  50
<211>  8
<212>  PRT
<213>  Artificial Sequence

<220>
<223>  fibril peptide 50

<400>  50

Arg Thr Ile Phe Ile Ile Ala Met
1               5


<210>  51
<211>  8
<212>  PRT
<213>  Artificial Sequence

<220>
<223>  fibril peptide 51

<400>  51

Arg Thr Ile Phe Ile Ile Ser Ala
1               5

<210>  52
<211>  7
<212>  PRT
<213>  Artificial Sequence

<220>
<223>  fibril peptide 52

<400>  52

Thr Ile Phe Ile Ile Ser Met
1               5


<210>  53
<211>  12
<212>  PRT
<213>  Artificial Sequence

<220>
<223>  Peptide RS-12

<400>  53

Arg Asp Asn Ala Pro Arg Thr Ile Phe Ile Ile Ser
1               5                   10


<210>  54
<211>  10
<212>  PRT
<213>  Artificial Sequence

<220>
<223>  Peptide RK-10

<400>  54

Arg Thr Ile Phe Ile Ile Ser Met Tyr Lys
1               5                   10


<210>  55
<211>  7
<212>  PRT
<213>  Artificial Sequence

<220>
<223>  Peptide RS-7

<400>  55

Arg Thr Ile Phe Ile Ile Ser
1               5


<210>  56
<211>  7
<212>  PRT
<213>  Artificial Sequence

<220>
<223>  Peptide FK-7



## Claims

1. A fibril peptide consisting of a sequence of from 8 to 20 amino acids and wherein said fibril peptide comprises the amino acid sequence RTIFIISM (SEQ ID NO:1) or a sequence at least 75% identical thereto.

2. The fibril peptide of claim 1, wherein said fibril peptide comprises the amino acid sequence X¹TIFIISM (SEQ ID NO:2), RX²IFIISM (SEQ ID NO:3), RTX²FIISM (SEQ ID NO:4), RTIX²IISM (SEQ ID NO:5), RTIFX²ISM (SEQ ID NO:6), RTIFIX²SM SEQ ID NO:7), RTIFIIX²M (SEQ ID NO:8), or RTIFIISX² (SEQ ID NO:9), wherein X¹ is a positively charged amino acid, preferably R, K or H, and wherein X² is any amino acid.

3. The fibril peptide of claim 1 or 2, wherein said fibril peptide comprises the amino acid sequence X¹TIFIISM (SEQ ID NO:2), RX²IFIISM (SEQ ID NO:3), RTX²FIISM (SEQ ID NO:4), RTIX²IISM (SEQ ID NO:5), RTIFX²ISM (SEQ ID NO:6), RTIFIX²SM SEQ ID NO:7), RTIFIIX²M (SEQ ID NO:8), or RTIFIISX² (SEQ ID NO:9), wherein X¹ is R or K and wherein X² is selected from the list consisting of A, V, L, I, M, F, T, and non-negatively charged derivatives thereof.

4. The fibril peptide of claim 3, wherein said derivative is a straight-chain amino acid, preferably homoalanine, norvaline, or norleucine; a D-amino acid, preferably D-alanine, D-leucine, or D-isoleucine; a non-alpha amino acid, preferably beta-alanine or gamma-amino-butyric acid; a derivative comprising an exchange of an atom by an atom from the same chemical group, preferably selenomethionine; and/or a naturally occurring derivative of an amino acid, preferably hypusine.

5. The fibril peptide of any one of claims 1 to 4, wherein said fibril peptide consists of the amino acid sequence RTIFIISMX³X⁴, RAIFIISMX³X⁴, RTAFIISMX³X⁴, RTIAIISMX³X⁴, RTIFAISMX³X⁴, RTIFIASMX³X⁴, RTIFIIAMX³X⁴, RTIFIISAX³X⁴, wherein X³ and X⁴ are independently selected from any amino acid or wherein X³ and/or X⁴ are absent.

6. The fibril peptide of any one of claims 1 to 5, wherein said fibril peptide consists of the amino acid sequence RTIFIISM, RAIFIISM, RTAFIISM, RTIAIISM, RTIFAISM, RTIFIASM, RTIFIIAM, RTIFIISA, RTIFIISMY, RAIFIISMY, RTAFIISMY, RTIAIISMY, RTIFAISMY, RTIFIASMY, RTIFIIAMY, RTIFIISAY, RTIFIISMA, RTIFIISMYK, RAIFIISMYK, RTAFIISMYK, RTIAIISMYK, RTIFAISMYK, RTIFIASMYK, RTIFIIAMYK, RTIFIISAYK, RTIFIISMAK, or RTIFIISMYA.

7. The fibril peptide of any one of claims 1 to 6, wherein said fibril peptide consists of the amino acid sequence RTIFIISM, RAIFIISM, RTAFIISM, RTIAIISM, RTIFAISM, RTIFIASM, RTIFIIAM, RTIFIISA, or RTIFIISMYK

8. The fibril peptide of any one of claims 1 to 7, wherein said fibril peptide has low immunogenicity, preferably is non-immunogenic to a mammal, preferably to a human.

9. A method of delivering a cargo comprised in a particle into a host cell, comprising
a) contacting said host cell with said particle and with a fibril peptide according to any one of claims 1 to 8; and, thereby
b) delivering said cargo into said host cell.

10. The method of claim 9, wherein said particle is a virus particle, a virus-like particle, or a liposome comprising at least one polypeptide of a virus.

11. The method of any one of claims 9 to 10, wherein said cargo comprised in said particle is a biological macromolecule or a pharmaceutical compound.

12. A fibril peptide according to any one of claims 1 to 8 for use in the treatment of disease, preferably for use in the treatment and/or prevention of cancer, genetic disease, and/or neurodegenerative disease.

13. Use, preferably in vitro use, of a fibril peptide according to any one of claims 1 to 8 for delivering a cargo comprised in a particle into a cell.

14. A kit comprising the fibril peptide according to any one of claims 1 to 8 and at least one solvent and/or diluent.

15. A device comprising the fibril peptide according to any one of claims 1 to 8.
